# EUROPEAN PATENT APPLICATION

(11) **EP 3 266 465 A1**
(43) Date of publication of application: **10.01.2018**
(21) Application number: 16178204.0
(22) Date of filing: 06.07.2016
(51) Int. Cl.: A61K 39/395, A61K 51/10, A61K 49/00, G01N 33/533, G01N 33/534

(54) **IMMUNE COMPLEXES**

(71) Applicant: Julius-Maximilians-Universität Würzburg, 97070 Würzburg (DE)
(72) Inventor: ALMANZAR, Dr. Giovanni, 97072 Würzburg (DE); BLEY, Prof. Dr. Thorsten, 97218 Gerbrunn (DE); GUTJAHR, Dr. Fabian, 97084 Würzburg (DE); JAKOB, Prof. Dr. Peter Michael, 97262 Würzburg (DE); KÖSTLER, Prof. Dr. Herbert, 97225 Zellingen (DE); KÜRTEN, Prof. Dr. Stefanie, 97070 Würzburg (DE); PRELOG, Prof. Dr. Martina, 97074 Würzburg (DE); WECH, Dr. rer. nat. Tobias, 97080 Würzburg (DE)
(74) Representative: Dr. Gassner & Partner mbB

(57) **Abstract**

The invention concerns immune complexes for use in a diagnostic method for detecting and localizing specific cells in a human or animal body by an imaging technique. First antibodies are directed to an antigen located on the surface of said cells. The first antibodies are optionally labeled with first labels. The method comprises that the first antibodies and second antibodies directed to said first antibodies or to the first labels or the first antibodies and ligands specifically binding to the first labels are administered to said human or animal body, wherein said second antibodies or said ligands are labeled with second labels detectable by said imaging technique. The cells are detected and localized in the human or animal body after administration of the first antibodies and second antibodies or of the first antibodies and the ligands by use of the imaging technique. The immune complexes of the invention are formed from the first antibodies and the second antibodies or from the first antibodies and the ligands ex *vivo* before administration of said immune complexes to the human or animal body.

## Description

The present invention concerns immune complexes for use in a diagnostic method, for use as a medicament and for use in a method of treatment.

Baio, G. et al., Mol. Imaging Biol. (2010) 12, pages 305 to 315 concerns a two-step *in vivo* tumor targeting by specific biotin-conjugated antibodies and ultrasmall superparamagnetic iron oxide (USPIO)-anti-biotin nanoparticles as contrast agents for magnetic resonance imaging (MRI) at 1.5 T (tesla). For demonstrating the functioning of the targeting human lymphoma cells expressing the CD70 surface antigen were injected either s.c. or i.v. to induce pseudo-metastases in NOD/SCID mice. Thirty micrograms of biotin-conjugated monoclonal anti-CD70 was injected i.v., followed 4 h later by 8 µmol Fe/kg USPIO-anti-biotin. After 24 h, MRI was performed on T2* and b-FFE sequences. Signal intensity (SI) was calculated before and after USPIO-anti-biotin administration. Subcutaneous xenografts showed a dishomogeneous 30% decrease in SI on T2* with anti-CD70+USPIO-anti-biotin treatment. Pseudo-metastatic xenografts showed a slight reduction in SI on T2*, but a 60% decrease in SI on b-FFE-weighted sequences. Prussian blue staining confirmed the presence of iron nanoparticles in the excised tumors. This shows that MRI at 1.5 T can detect tumors by a two-step *in vivo* biotin-based protocol, which may allow the targeting of any cell surface antigen. The document also discloses an *in vitro* MRI detection of cells labeled with biotin-labeled monoclonal anti-CD70 antibodies and USPIO-anti-biotin antibodies.

The problem to be solved by the present invention is to provide a detection tool allowing an improved selective imaging of target cells *in vivo.* A further problem to be solved by the present invention is to provide a product for use as a medicament and a product for use in a method of treatment of a disease.

The problems are solved by the subject-matter of claims 1, 11, 12 and 15. Embodiments of the invention are subject-matter of claims 2 to 10, 13 and 14.

According to the invention immune complexes are provided. The immune complexes are for use in a diagnostic method for detecting and localizing specific cells in a human or animal body by an imaging technique. First antibodies are directed to an antigen located on the surface of said cells. The first antibodies are optional-ly labeled with first labels. The method comprises that the first antibodies and second antibodies directed to said first antibodies or to the first labels or the first antibodies and ligands specifically binding to the first labels are administered to said human or animal body, wherein said second antibodies or said ligands are labeled with second labels detectable by said imaging technique. The cells are detected and localized in the human or animal body after administration of the first antibodies and second antibodies or of the first antibodies and the ligands by use of the imaging technique. The immune complexes of the invention are formed from the first antibodies and the second antibodies or from the first antibodies and the lig-ands *ex vivo* before administration of said immune complexes to the human or animal body.

The inventors of the present invention recognized that the forming of the immune complexes outside the human or animal body allows a more intense labeling of the cells to be detected, i. e. a higher density of the second label on the cells. This can be achieved by a higher ratio of the second antibodies to the first antibodies or of the ligands to the first antibodies in the immune complexes, in particular a ratio of at least 1 : 1. An even better labeling can be achieved if this ratio is at least 2 : 1, in particular at least 3 : 1, in particular at least 4 : 1, in particular at least 5 : 1. Such a ratio can be obtained, e. g. if the second antibodies are polyclonal antibodies recognizing a plurality of different epitopes on the first antibodies or the first labels. Providing immune complexes according to the invention with such a ratio results in a multiplication of the number of second labels localized on each cell to which one of the first antibodies is bound. The forming of the immune complexes *ex vivo* enables a forming of the complexes under defined conditions with defined concentrations of the first and the second antibodies or of the first antibodies and the ligands thus allowing the formation of complexes with a relatively high number of second labels. If the first antibodies are labeled with first labels there is usually at least one first label per first antibody. Usually there is also at least one second label per second antibody or per ligand. All of the first antibodies, the first labels, the second antibodies, the ligands and the second labels in each case may be identical. The first antibodies and/or the second antibodies in each case may be monoclonal or polyclonal antibodies.

In contrast to this the first antibodies are highly diluted when administered to the mice and only a part of the administered second USPIO-anti-biotin antibodies finds and binds to the first antibodies bound to the target cells in the method known from Baio et al.. This results in a low density of the USPIO-labels on the target cells and therewith to a low resolution when detecting the target cells in the animal body.

The higher the density of second labels on the specific cell is, the better is the resolution that can be achieved with the imaging technique. The high density of second labels provided by the immune complexes according to the invention allows a very precise localization of the specific cells to which the immune complexes are bound in the human or animal body. According to the present invention the imaging technique may be MRI and the second labels may be or may comprise paramagnetic iron oxide particles, in particular superparamagnetic iron oxide (SPIO) particles, in particular particles comprising or consisting of FeO-Fe₂O₃.

Alternatively, the imaging technique may be positron emission tomography (PET) and the second labels may be or may comprise positron-emitting radionuclides, in particular 2-deoxy-2-(¹⁸F)fluoro-D-glucose (¹⁸F-FDG). Alternatively, the imaging technique may be scintigraphy and the second labels may be or may comprise radionuclides. Any other imaging technique and corresponding labels may also be used.

The antigen may be any cell surface marker, in particular a marker for T cells, such as CD4 or CD8, a marker for B cells, such as CD19, or a marker for any other immune cells. The antigen may also be an immune cell differentiation marker, such as CD45RO or a migration factor, i. e. a chemokine receptor, such as CCR6. CD4 is a characteristic surface antigen of T helper cells. CCR6 is a CC chemokine receptor protein present on the surface of immature dendritic cells, B cells and memory T cells.

The cells to be detected and localized in the human or animal body may be isolated from the human or animal body prior to administration of the immune complexes according to the invention. After isolation the cells may be labeled with the immune complexes according to the invention or with the first antibodies and the second antibodies or with the first antibodies and the ligands such that the immune complexes are formed on said cells. Then the cells can be administered to the human or animal body together with the immune complexes bound to said cells.

Alternatively or additionally the diagnostic method comprises that the immune complexes are administered orally or injected directly in the human or animal body, in particular subcutaneously, intramuscularly or intradermally or in a vein of the human or animal body. Alternatively or additionally the complexes may be injected directly into the peritoneum of the human or animal body or in a structure of the human or animal body, which structure shall be examined, such as an inflamed joint or tissue. "Injected directly" means that the immune complexes are not bound to a carrier such as previously isolated cells of the human or animal body.

The cells may be any cells carrying said antigen as specific marker on the cell surface, in particular leucocytes, in particular chemokine receptor positive leucocytes, in particular CCR6 positive leucocytes.

Therapeutically active substances may be bound to the first antibodies and/or the second antibodies or to the first antibodies and/or the ligands. The therapeutically active substances may all be identical or may comprise different substances. The therapeutically active substances may be chemotherapeutic substances, in particular cytotoxic substances. In this way the immune complexes according to the invention allow a specific treatment of the cells to be detected and localized and in particular the killing of these specific cells, e. g. inflammatory cells or in the case of cancer cells of the cancer, such as a cancer of blood.

The first labels can comprise or consist of biotin, phycoerythrin (PE), any other label or the therapeutically active substance. PE is useful when the diagnostic method is performed in the animal body. When the first labels comprise or consist of biotin the ligands may be avidin molecules or streptavidin molecules.

The invention also concerns immune complexes as specified above for use as a medicament, e. g. for the treatment of a disease caused by said cells, such as an inflammation or a cancer, in particular a cancer of blood.

The invention also concerns immune complexes as specified above for use in a method of treatment of a disease caused by said cells, such as an inflammation or a cancer, in particular a cancer of blood.

The treatment may be a targeted radionuclide therapy and the second labels may be radionuclides. Alternatively, the treatment may be a treatment by use of an alternating magnetic or an alternating electric field resulting in a heating of the second labels in said field wherein the second labels are paramagnetic/the paramagnetic iron oxide particles, in particular superparamagnetic/the supermagnetic iron oxide (SPIO) particles, in particular particles/the particles comprising or consisting of FeO-Fe₂O₃. In case of an alternating electric field the second labels are heated by electromagnetic induction. In case of an alternating magnetic field the second labels are forced into an oscillation movement resulting in a heating. The heating is then used for a heat therapy. By use of the heat therapy the specific cells or the tissue in which the specific cells are located can be specifically damaged or destroyed.

The invention further concerns a use of the immune complexes as specified above in a non-therapeutic treatment in an animal model or in a non-diagnostic method for detecting and localizing specific cells in an animal model.

The invention is further illustrated on basis of the following embodiments.
- Fig. 1: shows a T2* map before (left hand side) and after (right hand side) administration of the immune complexes. A legend correlating gray scale values to T2* times is given on the outer right side of the figure.
- Fig. 2: shows MRI images of a mouse at different echo times in an animal after injection.
- Fig. 3a to 3d: show histograms of T2* times measured in the spleen of the animals before and after injection of the immune complexes according to the invention.
- Fig. 4a and 4b: show prussian blue stainings of histological sections of the spleen of an animal treated with the immune complexes (Fig. 4b) and an animal not treated with the immune complexes (Fig. 4a).

Immune complexes according to the invention were prepared by incubating 20 µL PE-labeled anti-CCR6-antibodies (Miltenyi Biotec GmbH, Bergisch-Gladbach, Germany) with 40 µL anti-PE-antibodies labeled with FeO-Fe₂O₃ MicroBeads (Miltenyi Biotec GmbH, Bergisch-Gladbach, Germany) in 160 µL MACS buffer (Miltenyi Biotec GmbH, Bergisch-Gladbach, Germany) for 15 min at 4 °C.

The spleen of two healthy C57BL/6 mice (animals 1 and 2) was imaged in vivo before and after administration of 100 µl of the immune complex via tail vain using MRI. All measurements were carried out on a 7T horizontal bore small animal system (Bruker Biospec, Bruker, Ettlingen, Germany) using a 35 mm quadrature birdcage. A multi echo gradient echo sequence was used to acquire images at 8 different echo times (TE) (TE: 2.13 ms, 6.13 ms, 10.13 ms, 14.13 ms, 18.13 ms, 22.13 ms, 26.13 ms, 30.13 ms).

Resolution parameters were 20 slices, 0.75 mm slice thickness, no gap between slices, field of view 4 x 3 cm², matrix size 256 x 192. This results in a total volume of (4.0 x 3.0 x 1.5) cm³ and an in-plane resolution of 156 µm x 156 µm. To minimize partial volume effects the imaging slices were orientated so that they are perpendicular to long axis of the spleen.

From the obtained images T2* times were determined for every voxel by fitting to the model a * exp(-TE/T2*). The spleen was manually selected in all slices. Afterwards the median of the values in the selected voxels was calculated.

The application of the immune complexes decreased the T2* times in spleen as follows:

**Table 1**

| | Animal 1 | Animal 2 |
|---|---|---|
| Before application of the immune complexes | 4.37 ms | 4.16 ms |
| After application of the immune complexes | 2.96 ms | 2.95 ms |
| Relative Shortening | 32% | 29% |

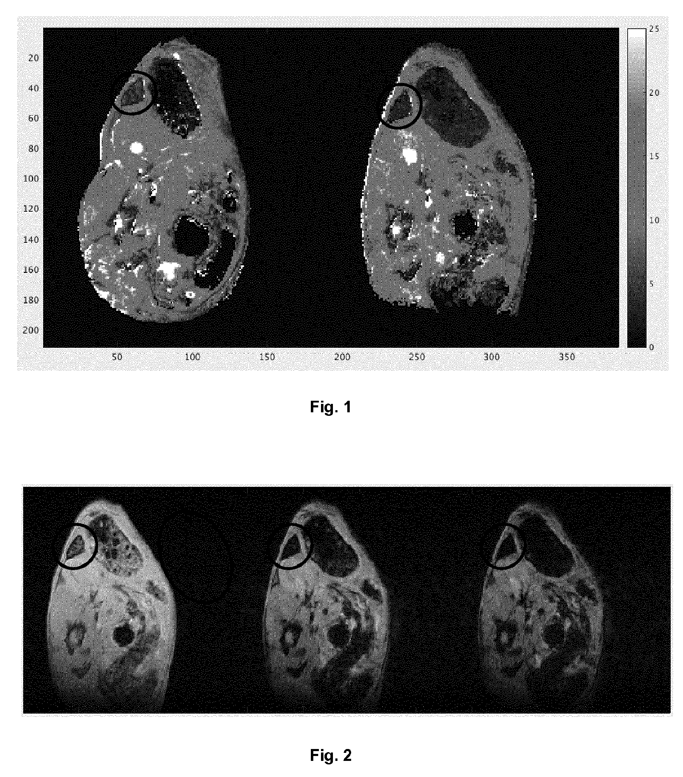

Fig. 1 shows a T2* map before (left hand side) and after (right hand side) administration of the immune complexes. A legend correlating gray scale values to T2* times is given on the outer right side of the figure. Fig. 1 and the above table show a clear negative contrasting of the spleen tissue by shortening the T2* relaxation times of the spleen due to the administration of the immune complexes. The spleen is indicated by a black circle.

Fig. 2 shows the first three echo images (TE: 2.13 ms, 6.13 ms, 10.13 ms, left to right) of a slice in the center of the spleen for animal 1 after administration of immune complexes. The signal of the spleen decays strongly between images, demonstrating the fast T2* relaxation. The spleen is indicated by a black circle.

Images obtained with animal 2 are not shown. They were very similar to those obtained with animal 1.

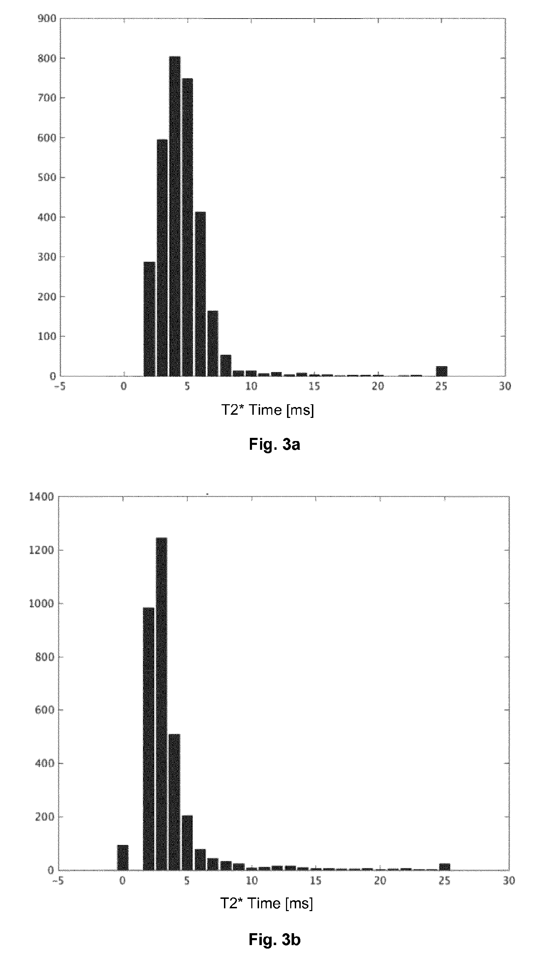

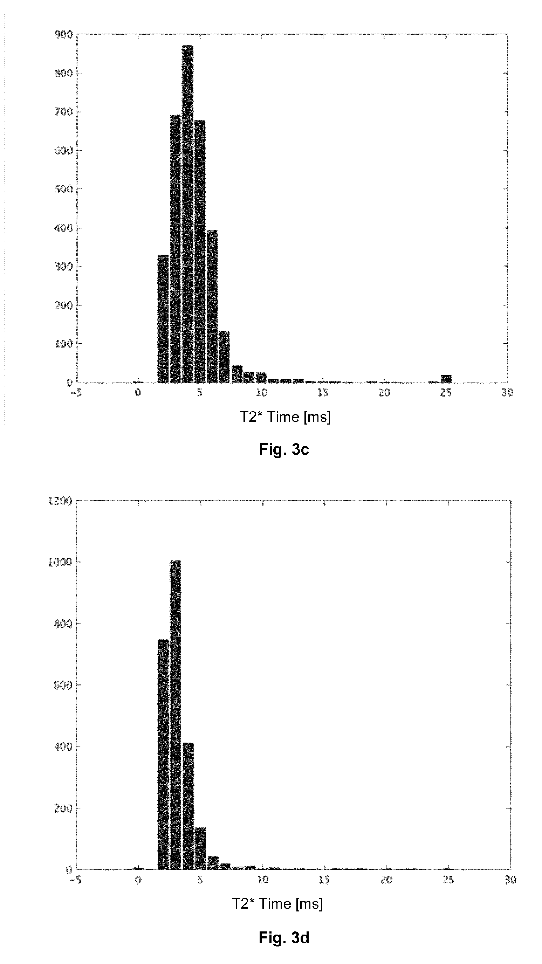

Fig. 3a to 3d show histograms of the measured relaxation times. The number of voxels is plotted against the relaxation times given on the x-axes. Fig. 3a shows the values obtained from the spleen tissue of animal 1 before administration of the immune complexes. The resulting median was 4.4 ms. Fig. 3b shows the values obtained from the spleen tissue of animal 1 after administration of the immune complexes. The resulting median was 3.0 ms. Fig. 3c shows the values obtained from the spleen tissue of animal 2 before administration of the immune complexes. The resulting median was 4.2 ms. Fig. 3d shows the values obtained from the spleen tissue of animal 2 after administration of the immune complexes. The resulting median was 2.9 ms.

Fig. 3a to 3d clearly show the shortening of the T2* relaxation times in the spleen tissues after application of the immune complexes according to the invention.

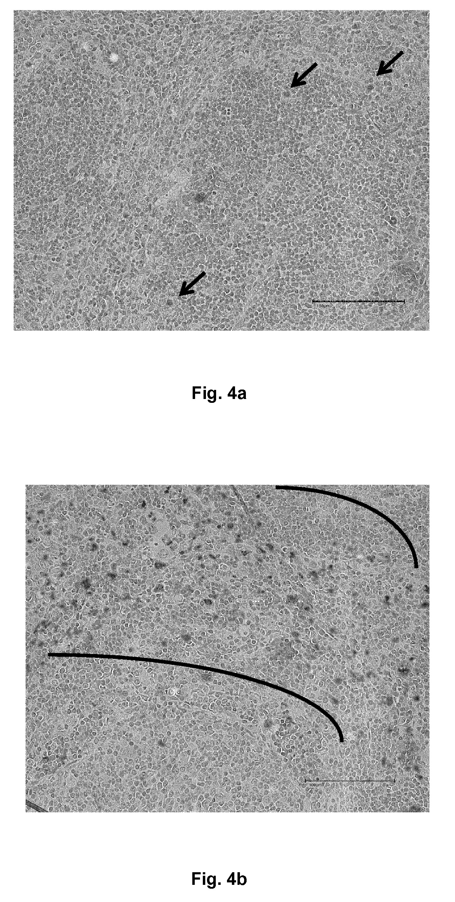

Animals were sacrified 24 hours after injection of immune complexes. Fig. 4b shows a Prussian blue staining (black cells in the gray-scale picture) of a histological section of the spleen confirming the presence of iron nanoparticles in the spleen in the perifollicular regions (area between black lines in Fig. 4b).

Fig. 4a shows the Prussian blue staining of a histological section of a representative spleen of an animal without administration of immune complexes used as a negative control. Only singular spleen cells physiologically storing iron particles were found in the negative control indicated by arrows.

## Claims

1. Immune complexes for use in a diagnostic method for detecting and localizing specific cells in a human or animal body by an imaging technique, wherein first antibodies are directed to an antigen located on the surface of said cells, wherein the first antibodies are optionally labeled with first labels, wherein said method comprises that the first antibodies and second antibodies directed to said first antibodies or to the first labels or the first antibodies and ligands specifically binding to the first labels are administered to said human or animal body, wherein said second antibodies or said ligands are labeled with second labels detectable by said imaging technique, wherein said cells are detected and localized in the human or animal body after administration of the first antibodies and second antibodies or of the first antibodies and the ligands by use of the imaging technique, **characterized in that** said immune complexes are formed from the first antibodies and the second antibodies or from the first antibodies and the ligands *ex vivo* before administration of said immune complexes to the human or animal body.

2. Immune complexes for use in a diagnostic method according to claim 1, wherein said imaging technique is positron emission tomography (PET) and said second labels are or comprise positron-emitting radionuclides, in particular 2-deoxy-2-(¹⁸F)fluoro-D-glucose (¹⁸F-FDG), or wherein said imaging technique is magnetic resonance imaging (MRI) and said second labels are or comprise paramagnetic iron oxide particles, in particular superparamagnetic iron oxide (SPIO) particles, in particular particles comprising or consisting of FeO-Fe₂O₃ or wherein said imaging technique is scintigraphy and said second labels are or comprise radionuclides.

3. Immune complexes for use in a diagnostic method according to any of the preceding claims, wherein said antigen is a cell surface marker, in particular a marker for immune cells, in particular a marker for T cells, in particular CD4 or CD8, or a marker for B cells, in particular CD19, an immune cell differentiation marker, in particular CD45RO, or a chemokine receptor, in particular CCR6.

4. Immune complexes for use in a diagnostic method according to any of the preceding claims, wherein the diagnostic method comprises that the immune complexes are administered orally or injected directly in the human or animal body, in particular subcutaneously, intramuscularly or intradermally or in a vein or in a peritoneum of the human or animal body or in a structure of the human or animal body, which structure shall be examined, in particular in an inflamed joint or tissue.

5. Immune complexes for use in a diagnostic method according to any of the preceding claims, wherein the diagnostic method comprises that said cells are isolated from the human or animal body prior to administration of said immune complexes, then labeled with said immune complexes or with the first antibodies and the second antibodies or with the first antibodies and the ligands such that the immune complexes are formed on said cells and then administered to the human or animal body together with the immune complexes bound to said cells.

6. Immune complexes for use in a diagnostic method according to any of the preceding claims, wherein said cells are leucocytes, in particular chemokine receptor positive leucocytes, in particular CCR6 positive leucocytes.

7. Immune complexes for use in a diagnostic method according to any of the preceding claims, wherein a ratio of said second antibodies to said first antibodies or of the said ligands to said first antibodies in the immune complexes is at least 2 : 1, at least 3 : 1, at least 4 : 1, or at least 5 : 1.

8. Immune complexes for use in a diagnostic method according to any of the preceding claims, wherein therapeutically active substances are bound to the first antibodies and/or the second antibodies or to the first antibodies and/or the ligands.

9. Immune complexes for use in a diagnostic method according to any of the preceding claims, wherein said first labels comprise or consist of biotin, phycoerythrin (PE), or the therapeutically active substance.

10. Immune complexes for use in a diagnostic method according to claim 9, wherein the first labels comprise or consist of biotin, wherein the ligands are avidin molecules or streptavidin molecules.

11. Immune complexes as specified in any of the preceding claims for use as a medicament.

12. Immune complexes as specified in any of claims 1 to 10 for use in a method of treatment of a disease caused by said cells.

13. Immune complexes for use in a method of treatment according to claim 12, wherein the disease is an inflammation or a cancer, in particular a cancer of blood.

14. Immune complexes for use in a method of treatment according to claim 12 or 13, wherein the treatment is a targeted radionuclide therapy and the second labels are radionuclides or wherein the treatment is a treatment by use of an alternating magnetic or an alternating electric field resulting in a heating of the second labels in said field, wherein the second labels are paramagnetic/the paramagnetic iron oxide particles, in particular superparamagnetic/the superparamagnetic iron oxide (SPIO) particles, in particular particles/the particles comprising or consisting of FeO-Fe₂O₃.

15. Use of the immune complexes as specified in any of claims 1 to 10 in a non-therapeutic treatment in an animal model or in a non-diagnostic method for detecting and localizing specific cells in an animal model.
